# EUROPEAN PATENT APPLICATION

(11) **EP 4 834 904 A1**
(43) Date of publication of application: **04.11.2026**
(21) Application number: 24912817.4
(22) Date of filing: 24.12.2024
(51) Int. Cl.: C07D 471/16, B01J 31/02, B01J 35/39, C07B 61/00, C07C 67/31, C07C 69/78

(54) **POLYCYCLIC COMPOUND, METHOD FOR REDUCING ORGANIC COMPOUND, AND METHOD FOR COUPLING ORGANIC COMPOUND**

(30) Priority: 25.12.2023 JP 2023217785
(71) Applicant: Inter-University Research Institute Corporation National Institutes of Natural Sciences, Tokyo 181-8588 (JP)
(72) Inventor: OKUMURA Shintaro, Okazaki-shi, Aichi 444-8787 (JP); UOZUMI Yasuhiro, Okazaki-shi, Aichi 444-8787 (JP)
(74) Representative: Laine IP Oy
(86) International application number: PCT/JP2024/045703
(87) International publication number: WO 2025/142930

(57) **Abstract**

A polycyclic compound applicable to a reduction reaction or a coupling reaction of an organic compound, using visible light as an energy source; and a polycyclic compound applicable to a fluorescent material are provided. The polycyclic compound according to the present invention is represented by Formula (1a) or Formula (1b).

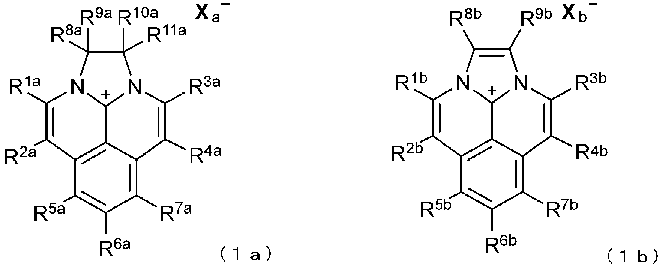

In Formula (1a) or Formula (1b), R^{1a} to R^{11a} and R^{1b} to R^{9b} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and Xₐ⁻ and X_{b}⁻ are each a counter anion.

## Description

### TECHNICAL FIELD

The present invention relates to a polycyclic compound, a method for reducing an organic compound, and a method for coupling organic compounds.

Priority is claimed on Japanese Patent Application No. 2023-217785, filed December 25, 2023, the content of which is incorporated herein by reference.

### BACKGROUND ART

Photocatalysts are used for reduction reactions, coupling reactions and the like of organic compounds such as carbonyl compounds.

As the photocatalyst, for example, iridium-based catalysts, ruthenium-based catalysts, and the like are known (for example, Non Patent Document 1).

### Citation List

### Non Patent Document

Non Patent Document 1: David W. C. MacMillan, et al., "Chem. Rev.", 2013, Vol. 113, No. 7, p. 5322 to 5363

### SUMMARY OF INVENTION

### Technical Problem

Photocatalysts in the related art have had limitations in the reduction reaction or coupling reaction under mild conditions such as using visible light as an energy source. Therefore, the photocatalysts in the related art are not suitable for the reduction reaction or coupling reaction of the organic compound, using visible light as an energy source.

An objective of the present invention is to provide a novel compound applicable to a reduction reaction or a coupling reaction of an organic compound, using visible light as an energy source.

Another objective of the present invention is to provide a novel compound applicable to a fluorescent material.

### Solution to Problem

The present invention has the following aspects.
[1] A polycyclic compound represented by Formula (1a) or Formula (1b). 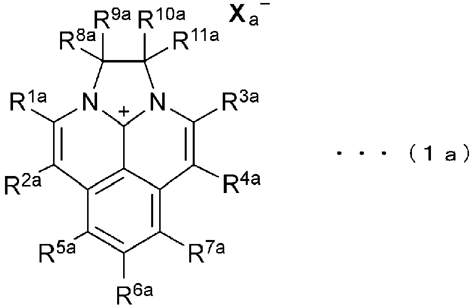 In Formula (1a), R^{1a} to R^{11a} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and Xₐ⁻ is a counter anion. 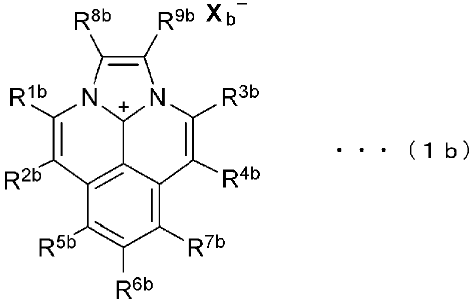 In Formula (1b), R^{1b} to R^{9b} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and X_{b}⁻ is a counter anion.
[2] The polycyclic compound according to [1],
   in which, in Formula (1a), R^{1a} to R^{4a} are each independently a hydrocarbon group having 1 to 20 carbon atoms and optionally containing a hetero atom, R^{6a} is a hydrogen atom, a hydroxy group, a hydrocarbonoxy group having 1 to 20 carbon atoms, or a dialkylamino group having 2 to 21 carbon atoms, and R^{5a}, R^{7a}, and R^{8a} to R^{11a} are each a hydrogen atom, and
   in Formula (1b), R^{1b} to R^{4b} are each independently a hydrocarbon group having 1 to 20 carbon atoms and optionally containing a hetero atom, R^{6b} is a hydrogen atom, a hydroxy group, a hydrocarbonoxy group having 1 to 20 carbon atoms, or a dialkylamino group having 2 to 21 carbon atoms, and R^{5b}, R^{7b}, R^{8b}, and R^{9b} are each a hydrogen atom.
[3] The polycyclic compound according to [2],
   in which R^{1a} to R^{4a} in Formula (1a) are each independently an alkyl group having 1 to 20 carbon atoms and optionally containing a hetero atom or an aralkyl group having 1 to 20 carbon atoms and optionally containing a hetero atom, and
   R^{1b} to R^{4b} in Formula (1b) are each independently an alkyl group having 1 to 20 carbon atoms and optionally containing a hetero atom or an aralkyl group having 1 to 20 carbon atoms and optionally containing a hetero atom.
[4] The polycyclic compound according to [3],
   in which R^{1a} to R^{4a} in Formula (1a) are each independently an alkyl group having 1 to 10 carbon atoms and containing no hetero atom, and
   R^{1b} to R^{4b} in Formula (1b) are each independently an alkyl group having 1 to 10 carbon atoms and containing no hetero atom.
[5] The polycyclic compound according to any one of [1] to [4],
   in which the polycyclic compound is a photocatalyst.
[6] The polycyclic compound according to any one of [1] to [4],
   in which the polycyclic compound is a fluorescent material.
[7] A method for reducing an organic compound, including reducing an organic compound using the polycyclic compound according to any one of [1] to [4].
[8] A method for coupling organic compounds, including coupling organic compounds using the polycyclic compound according to any one of [1] to [4].

### Advantageous Effects of Invention

According to the present invention, it is possible to provide a polycyclic compound applicable to a reduction reaction or a coupling reaction of an organic compound, using visible light as an energy source.

In addition, according to the present invention, it is possible to provide a polycyclic compound applicable to a fluorescent material.

### BRIEF DESCRIPTION OF DRAWINGS

[FIG. 1] An ultraviolet-visible absorption spectrum of a compound (1a-1) obtained in Example A1.
[FIG. 2] A fluorescence spectrum of the compound (1a-1) obtained in Example A1.

### DESCRIPTION OF EMBODIMENTS

Hereinafter, embodiments of the present invention will be specifically described.

However, the present invention is not limited to the following embodiments, and can be implemented with various modifications within the scope of the gist of the present invention.

In the present specification, a numerical range represented by "to" means a numerical range including numerical values before and after "to" as a lower limit value and an upper limit value. For example, A to B has the same meaning as A or more and B or less.

In the present specification, a methyl group is also referred to as "Me"; an ethyl group is also referred to as "Et"; a n-butyl group is also referred to as "Bu"; and an acetyl group is also referred to as "Ac".

### [Polycyclic compound]

A polycyclic compound according to an embodiment of the present invention is a cationic polycyclic compound having a hetero atom, which is represented by Formula (1a) or Formula (1b). In the present invention, a polycyclic compound represented by Formula (1a) is also referred to as "compound (1a)"; and a polycyclic compound represented by Formula (1b) is also referred to as "compound (1b)". In addition, the compound (1a) and the compound (1b) are collectively referred to as "compound (1)".

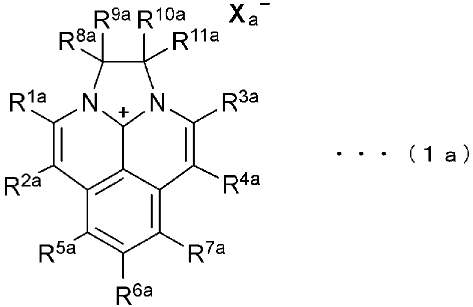

In Formula (1a), R^{1a} to R^{11a} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and Xₐ⁻ is a counter anion.

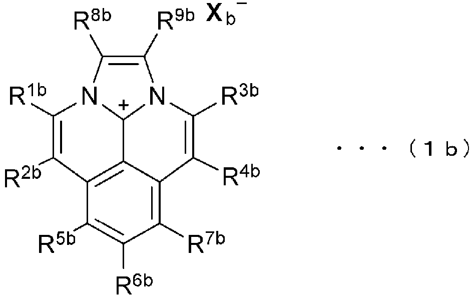

In Formula (1b), R^{1b} to R^{9b} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and X_{b}⁻ is a counter anion.

In Formula (1a), R^{1a} to R^{11a} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group.

In General Formula (1b), R^{1b} to R^{9b} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group.

The number of carbon atoms in the hydrocarbon group as R^{1a} to R^{11a} and R^{1b} to R^{9b} is 1 to 20, preferably 1 to 16, more preferably 1 to 12, still more preferably 1 to 10, even more preferably 1 to 8, especially preferably 2 to 8, and particularly preferably 2 to 5.

Examples of the hydrocarbon group include an alkyl group, an aryl group, and an aralkyl group; an alkyl group or an aryl group is preferable, and an alkyl group is more preferable.

The hydrocarbon group may have a substituent. Examples of the substituent include a carboxy group, a methoxycarbonyl group, an ethoxycarbonyl group, a butoxycarbonyl group, a cyano group, a hydroxy group, an amino group, an amide group, a halogen atom, and a phenyl group. The number of carbon atoms in the substituent is included in the number of carbon atoms in the hydrocarbon group described above.

The alkyl group may be linear, branched, or cyclic. Examples of the alkyl group include linear or branched alkyl groups such as a methyl group, an ethyl group, a n-propyl group, an i-propyl group, a n-butyl group, a t-butyl group, an i-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an icosyl group; and cycloalkyl groups such as a cyclopropyl group, a cyclobutyl group, a cyclopentyl group, a cyclohexyl group, a cycloheptyl group, a cyclooctyl group, and an adamantyl group.

Examples of the aryl group include a phenyl group, a tolyl group, and a xylyl group.

Examples of the aralkyl group include those in which one of hydrogen atoms of the alkyl group is substituted by an aryl group; and examples thereof include a benzyl group and a phenethyl group.

The hydrocarbon group may contain a hetero atom. The hetero atom is preferably contained between carbon atoms.

Examples of the hetero atom include a nitrogen atom, an oxygen atom, and a sulfur atom.

The hydrocarbon group is preferably an alkyl group containing no hetero atom. The number of carbon atoms in the alkyl group is preferably 1 to 16, more preferably 1 to 12, still more preferably 1 to 10, even more preferably 1 to 8, especially preferably 2 to 8, and particularly preferably 2 to 5.

The number of carbon atoms in the hydrocarbonoxy group as R^{1a} to R^{11a} and R^{1b} to R^{9b} is 1 to 20, preferably 1 to 10, more preferably 1 to 6, and still more preferably 1 to 5.

Examples of the hydrocarbonoxy group include an alkoxy group, an alkenyloxy group, and an aryloxy group; and an alkoxy group is preferable.

An alkyl group bonded to an oxygen atom in the alkoxy group may be linear, branched, or cyclic. The alkyl group may have a substituent. Examples of the substituent include a carboxy group, a methoxycarbonyl group, an ethoxycarbonyl group, a butoxycarbonyl group, a cyano group, a hydroxy group, an amino group, an amide group, a halogen atom, and a phenyl group. The number of carbon atoms in the substituent is included in the number of carbon atoms in the alkoxy group described above.

Examples of the alkoxy group include a methoxy group, an ethoxy group, a propoxy group, a butoxy group, and a cyclohexyloxy group; a methoxy group, an ethoxy group, a propoxy group, or a butoxy group is preferable, a methoxy group or an ethoxy group is more preferable, and a methoxy group is still more preferable. These groups may have a substituent.

Examples of the alkenyloxy group include a vinyloxy group, an isopropenyloxy group, and an allyloxy group; and an allyloxy group is preferable.

Examples of the aryloxy group include a phenoxy group and a naphthyloxy group.

The number of carbon atoms in the monoalkylamino group as R^{1a} to R^{11a} and R^{1b} to R^{9b} is 1 to 20, preferably 1 to 10 and more preferably 1 to 4.

An alkyl group in the monoalkylamino group may be linear, branched, or cyclic. The alkyl group may have a substituent. Examples of the substituent include those mentioned above in the description of the alkoxy group. The number of carbon atoms in the substituent is included in the number of carbon atoms in the monoalkylamino group described above.

Examples of the monoalkylamino group include a methylamino group, an ethylamino group, and a butylamino group; a methylamino group or an ethylamino group is preferable, and a methylamino group is more preferable.

The number of carbon atoms in the dialkylamino group as R^{1a} to R^{11a} and R^{1b} to R^{9b} is 2 to 21, preferably 2 to 12 and more preferably 2 to 8. The number of carbon atoms in the dialkylamino group is the total number of all carbon atoms contained in the dialkylamino group.

Each of alkyl groups in the dialkylamino group may be linear, branched, or cyclic. In addition, two alkyl groups substituted on an amino group of the dialkylamino group may be directly bonded to each other, or may be bonded to each other through an oxygen atom, a sulfur atom, or a nitrogen atom which may be substituted with an alkyl group having 1 to 6 carbon atoms, to form a ring. The alkyl group may have a substituent. Examples of the substituent include those mentioned above in the description of the alkoxy group. The number of carbon atoms in the substituent is included in the number of carbon atoms in the dialkylamino group described above. The two alkyl groups may be the same as or different from each other, and the substituents on the alkyl groups may be the same as or different from each other.

Examples of the dialkylamino group include a dimethylamino group, a methylethylamino group, a diethylamino group, a dipropylamino group, a dibutylamino group, a piperidinyl group, a pyrrolidinyl group, a morpholinyl group, and a piperazinyl group; a dimethylamino group, a methylethylamino group, a diethylamino group, a dipropylamino group, or a dibutylamino group is preferable, a dimethylamino group, a methylethylamino group, or a diethylamino group is more preferable, a dimethylamino group or a diethylamino group is still more preferable, and a dimethylamino group is particularly preferable.

The ester group as R^{1a} to R^{11a} and R^{1b} to R^{9b} refers to a functional group in which the aforementioned hydrocarbon group is bonded through an ester bond.

The number of carbon atoms in the ester group is preferably 1 to 20, more preferably 1 to 12, and still more preferably 1 to 6. The number of carbon atoms in the ester group includes the number of carbon atoms of the hydrocarbon group bonded through an ester bond and the substituent on the hydrocarbon group.

R^{1a} to R^{4a} in Formula (1a) and R^{1b} to R^{4b} in Formula (1b) may be each independently a hydrocarbon group having 1 to 20 carbon atoms and optionally containing a hetero atom; may be an alkyl group having 1 to 20 carbon atoms and optionally containing a hetero atom or an aralkyl group having 1 to 20 carbon atoms and optionally containing a hetero atom; may be an alkyl group having 1 to 20 carbon atoms and optionally containing a hetero atom; may be an alkyl group having 1 to 20 carbon atoms and containing no hetero atom; may be an alkyl group having 1 to 10 carbon atoms and optionally containing a hetero atom or an aralkyl group having 1 to 10 carbon atoms and optionally containing a hetero atom; may be an alkyl group having 1 to 10 carbon atoms and optionally containing a hetero atom; may be an alkyl group having 1 to 10 carbon atoms and containing no hetero atom; may be an alkyl group having 2 to 8 carbon atoms and optionally containing a hetero atom or an aralkyl group having 2 to 8 carbon atoms and optionally containing a hetero atom; may be an alkyl group having 2 to 8 carbon atoms and optionally containing a hetero atom; may be an alkyl group having 2 to 8 carbon atoms and containing no hetero atom; or may be an ethyl group, a n-propyl group, a n-butyl group, a n-pentyl group, a n-hexyl group, or a n-heptyl group. In addition, they may be an alkyl group having 2 to 5 carbon atoms and containing no hetero atom.

R^{1a} to R^{4a} in Formula (1a) may be the same as or different from each other. From the viewpoint of easy production, they are preferably the same as each other.

R^{1b} to R^{4b} in Formula (1b) may be the same as or different from each other. From the viewpoint of easy production, they are preferably the same as each other.

R^{5a} and R^{7a} in Formula (1a), and R^{5b} and R^{7b} in Formula (1b) are each preferably a hydrogen atom.

R^{5a} and R^{7a} in Formula (1a) may be the same as or different from each other. From the viewpoint of easy production, they are preferably the same as each other.

R^{5b} and R^{7b} in Formula (1b) may be the same as or different from each other. From the viewpoint of easy production, they are preferably the same as each other.

R^{6d} in Formula (1a) and R^{6b} in Formula (1b) are each independently preferably a hydrogen atom, a hydroxy group, an alkoxy group having 1 to 20 carbon atoms, or a dialkylamino group having 2 to 21 carbon atoms; more preferably a hydrogen atom, a hydroxy group, an alkoxy group having 1 to 6 carbon atoms, or a dialkylamino group having 2 to 8 carbon atoms; and still more preferably a hydrogen atom, a hydroxy group, a methoxy group, an ethoxy group, a propoxy group, a dimethylamino group, a diethylamino group, a dipropylamino group, or a dibutylamino group. An alkyl group in the alkoxy group and the dialkylamino group may have a substituent; and when it has a substituent, it is preferably a carboxy group, a methoxycarbonyl group, an ethoxycarbonyl group, a butoxycarbonyl group, a hydroxy group, a methoxy group, or an ethoxy group, and more preferably a carboxy group, a methoxycarbonyl group, an ethoxycarbonyl group, or a butoxycarbonyl group.

R^{6a} in Formula (1a) and R^{6b} in Formula (1b) may be each independently a hydrogen atom, a hydroxy group, a methoxy group, a dimethylamino group, a 3-methoxycarbonylpropyloxy group, or a 3-carboxypropyloxy group.

R^{8a} to R^{11a} in Formula (1a), and R^{8b} and R^{9b} in Formula (1b) are each preferably a hydrogen atom.

R^{8a} to R^{11a} in Formula (1a) may be the same as or different from each other. From the viewpoint of easy production, they are preferably the same as each other.

R^{8b} and R^{9b} in Formula (1b) may be the same as or different from each other. From the viewpoint of easy production, they are preferably the same as each other.

In Formula (1a), Xₐ⁻ is a counter anion.

In Formula (1b), X_{b}⁻ is a counter anion.

Examples of the counter anion include Cl⁻, Br⁻, I⁻, ClO₄⁻, BF₄⁻, PF₆⁻, CH₃COO⁻, CF₃SO₃⁻, CH₃SO₃⁻, p-CH₃C₆H₄SO₃⁻, F⁻, and SbF₆⁻; Cl⁻, BF₄⁻, CH₃COO⁻, or CF₃SO₃⁻ is preferable.

The compound (1a) includes, but is not limited to, compounds represented by Formulae (1a-1) to (1a-12).

The compound (1b) includes, but is not limited to, compounds represented by Formulae (1b-1) to (1b-12).

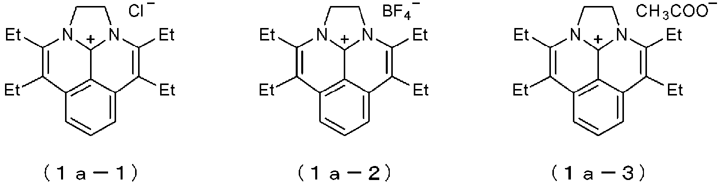

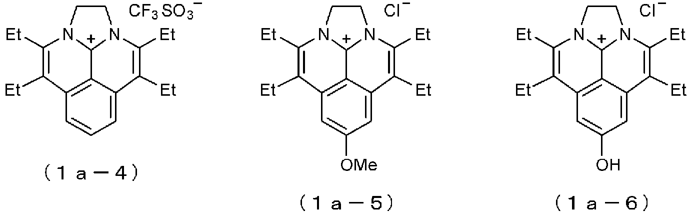

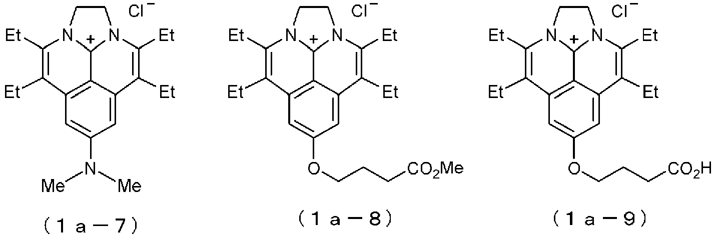

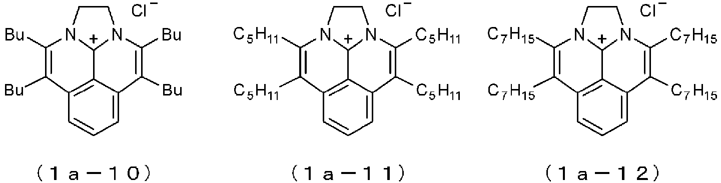

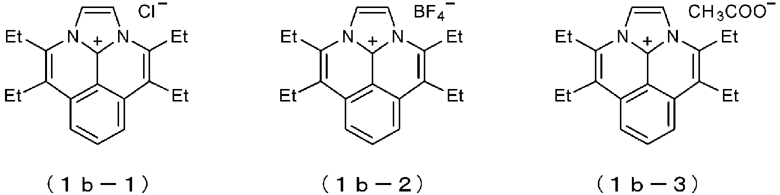

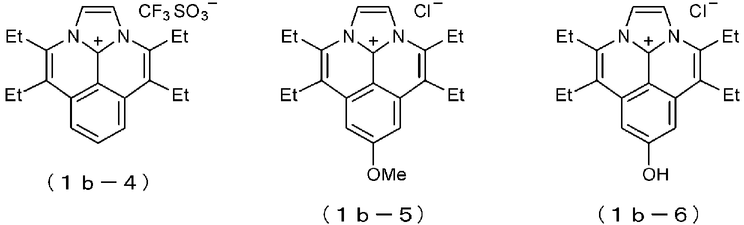

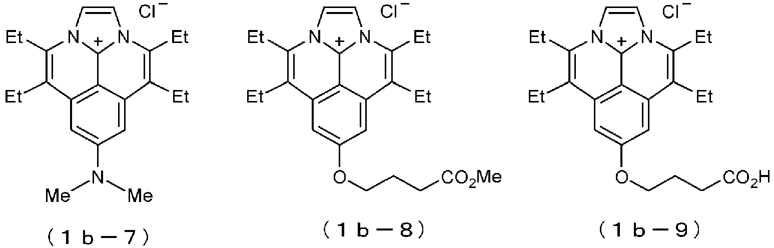

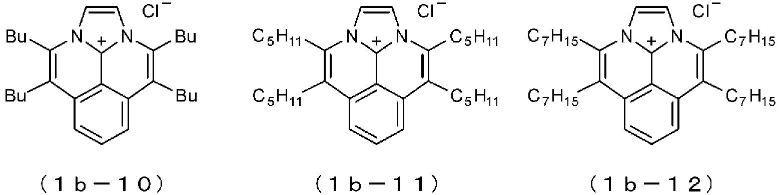

The compound (1a) can be produced, for example, as follows.

A compound (2a) of the following formula, a compound (31a) of the following formula, and a compound (32a) of the following formula are reacted in a solvent in the presence of a catalyst and an oxidizing agent to obtain the compound (1a).

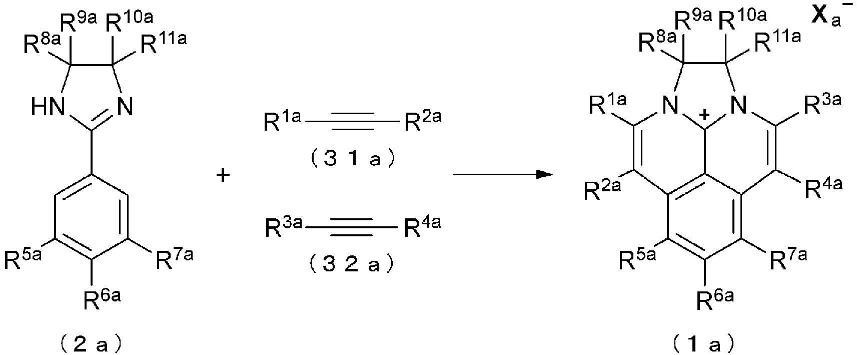

The compound (2a) may be synthesized by a known method, or a commercially available product thereof may be used.

Each of the compound (31a) and the compound (32a) may be synthesized by a known method, or a commercially available product thereof may be used.

The compound (31a) and the compound (32a) may be the same compound or different compounds.

A reaction proportion of the compound (2a) to the compound (31a) and the compound (32a) is such that the total amount of the compound (31a) and the compound (32a) is preferably 1 to 5 equivalents, more preferably 1 to 3 equivalents, and still more preferably 2.5 equivalents with respect to the compound (2a).

When the compound (31a) and the compound (32a) are different compounds, the amount of the compound (32a) is preferably 0.5 to 1.5 equivalents, more preferably 0.8 to 1.2 equivalents, and still more preferably 1 equivalent with respect to the compound (31a).

Examples of the catalyst include dichloro(pentamethylcyclopentadienyl)rhodium(11I) (dimer) ([Rh(Cp*)Cl₂]₂) and diacetoxy(pentamethylcyclopentadienyl)rhodium(III) (Rh(Cp*)(OAc)₂).

The catalyst may be used alone or in combination of two or more kinds at an arbitrary ratio.

A proportion of the catalyst is preferably 0.01 to 0.05 equivalents, more preferably 0.02 to 0.04 equivalents, and still more preferably 0.03 equivalents with respect to the compound (2a).

Examples of the oxidizing agent include silver acetate (AgOAc), copper acetate (Cu(OAc)₂), and hydrogen peroxide (H₂O₂).

The oxidizing agent may be used alone or in combination of two or more kinds at an arbitrary ratio.

A proportion of the oxidizing agent is preferably 2 to 10 equivalents, more preferably 3 to 6 equivalents, and still more preferably 4 equivalents with respect to the compound (2a).

Examples of the solvent include alcohol solvents such as methanol, ethanol, and isopropyl alcohol; saturated aliphatic hydrocarbon solvents such as hexane; ether solvents such as tetrahydrofuran, diethyl ether, cyclopentyl methyl ether, and methyl t-butyl ether; aromatic hydrocarbon solvents such as toluene and xylene; and nitrile solvents such as acetonitrile and benzonitrile.

The solvent may be used alone or in combination of two or more kinds at an arbitrary ratio.

Reaction conditions can be appropriately set depending on chemical properties and/or physical properties of the substrate.

A reaction temperature is preferably 40°C to 110°C, and more preferably 80°C to 90°C.

A reaction time is preferably 12 to 72 hours, and more preferably 15 to 24 hours.

The reaction between the compound (2a), and the compound (31a) and the compound (32a) is preferably carried out under a nitrogen atmosphere.

For example, when silver acetate is used as the oxidizing agent, the compound (1a) in which Xₐ⁻ in Formula (1a) is CH₃COO⁻ is obtained. By adding a chloride such as sodium chloride, potassium chloride, magnesium chloride, or calcium chloride to a reaction solution containing the compound (1a) as a reaction product, the compound (1a) in which Xₐ⁻ is Cl⁻ is obtained. Exchange of the counter anion can be performed by the same method.

The same applies when producing the compound (1b).

The compound (1b) can be produced, for example, as follows.

A compound (2b) of the following formula, a compound (31b) of the following formula, and a compound (32b) of the following formula are reacted in a solvent in the presence of a catalyst and an oxidizing agent to obtain the compound (1b).

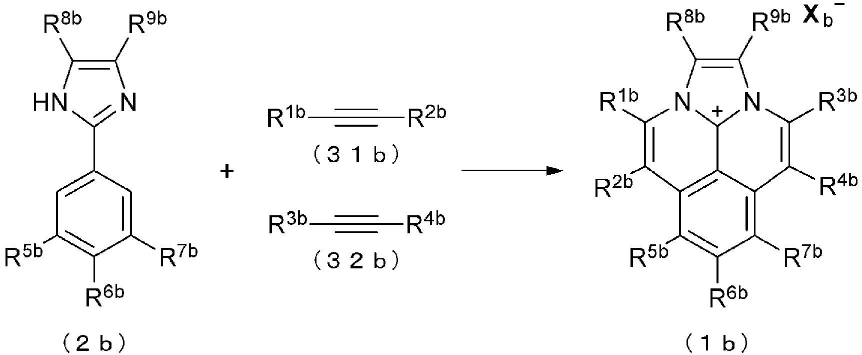

The compound (2b) may be synthesized by a known method, or a commercially available product thereof may be used.

Each of the compound (31b) and the compound (32b) may be synthesized by a known method, or a commercially available product thereof may be used.

The compound (31b) and the compound (32b) may be the same compound or different compounds.

A reaction proportion of the compound (2b) to the compound (31b) and the compound (32b) is such that the total amount of the compound (31b) and the compound (32b) is preferably 1 to 5 equivalents, more preferably 1 to 3 equivalents, and still more preferably 2.5 equivalents with respect to the compound (2b).

When the compound (31b) and the compound (32b) are different compounds, the amount of the compound (32b) is preferably 0.5 to 1.5 equivalents, more preferably 0.8 to 1.2 equivalents, and still more preferably 1 equivalent with respect to the compound (31b).

The catalyst, the oxidizing agent, the solvent, and the proportions thereof are the same as those when producing the compound (1a).

The reaction temperature and the reaction time are the same as those when producing the compound (1a).

The reaction between the compound (2b), and the compound (31b) and the compound (32b) is preferably carried out under a nitrogen atmosphere.

The polycyclic compound according to the embodiment of the present invention described above can absorb ultraviolet-visible light, can use the absorbed light as an energy source, and has high oxidizing power and high reducing power, and thus is optimal as a photocatalyst. That is, the polycyclic compound according to the embodiment of the present invention can also be referred to as a cationic organic photocatalyst.

The polycyclic compound according to the embodiment of the present invention can be used as a photocatalyst used in various reactions, but is suitable as a photocatalyst used in a reduction reaction or a coupling reaction of an organic compound. In particular, since the polycyclic compound according to the embodiment of the present invention can use visible light as an energy source, it is optimal as a photocatalyst applicable to a reduction reaction or a coupling reaction of an organic compound, using visible light as an energy source. In addition, since the polycyclic compound according to the embodiment of the present invention is soluble in water or an organic solvent, the reduction reaction, the coupling reaction, or the like can be carried out in water, in an organic solvent, or in a mixed solvent of water and an organic solvent.

The reduction reaction and the coupling reaction using the polycyclic compound according to the embodiment of the present invention will be described later.

Since the polycyclic compound according to the embodiment of the present invention can emit the absorbed light again as light, it is also suitable as a fluorescent material. Specifically, it is suitable as a fluorescent material for an organic EL element, a wavelength conversion film, a fluorescent paint, and the like.

### [Reduction method]

A method for reducing an organic compound according to an embodiment of the present invention (hereinafter, also referred to as "reduction reaction") is a method of reducing an organic compound in the presence of the polycyclic compound according to the embodiment of the present invention described above.

Examples of the organic compound suitable for the reduction method according to the embodiment of the present invention include a compound having a carbonyl group (hereinafter, also referred to as "carbonyl compound"; including an ester compound, an aldehyde compound, a ketone compound, and a carboxylic acid compound) and an imine compound. The reduction method according to the embodiment of the present invention is suitable as a reduction method of reducing the carbonyl compound, and more suitable as a reduction method of reducing an ester compound. In addition, the reduction method according to the embodiment of the present invention is suitable as a reduction method of reducing an aromatic carbonyl compound, and more suitable as a reduction method of reducing an aromatic ester compound.

In the reduction method according to the embodiment of the present invention, a concentration of the organic compound in 100% by mass of a reaction solution is preferably 0.001% to 10% by mass, more preferably 0.001% to 1% by mass, and still more preferably 0.01% to 0.1% by mass.

A proportion of the polycyclic compound is preferably 0.001 to 0.2 equivalents, more preferably 0.005 to 0.1 equivalents, and still more preferably 0.01 to 0.05 equivalents with respect to the organic compound.

As described above, since the polycyclic compound according to the embodiment of the present invention can absorb ultraviolet-visible light and can use the absorbed light as an energy source, the reduction reaction of the organic compound is preferably carried out while irradiating ultraviolet rays or visible light, and more preferably carried out while irradiating visible light.

Visible light is light having a wavelength of 380 to 780 nm. Examples of the visible light include light with a wavelength corresponding to a color such as blue, green, yellow, and red; in particular, light having a wavelength of 380 to 500 nm, so-called blue light, is suitable from the viewpoint of versatility.

An irradiation amount of the visible light (integrated light amount) is preferably 1000 to 100000 J/cm², more preferably 3500 to 50000 J/cm², and still more preferably 7000 to 30000 J/cm².

An output of the visible light may be set such that the irradiation amount falls within the above-described range, and varies depending on the size of the device and the irradiation range, but is, for example, preferably 1 to 1,000 W, more preferably 5 to 500 W, and still more preferably 10 to 100 W.

The reduction reaction is usually carried out in the presence of a reducing agent.

The reducing agent is not particularly limited, and examples thereof include oxalic acid, ammonium oxalate, sodium oxalate, sodium ascorbate, triethylamine, tributylamine, triethanolamine, and diisopropylethylamine.

The reducing agent may be used alone or in combination of two or more kinds at an arbitrary ratio.

Meanwhile, since oxalic acid, ammonium oxalate, and sodium oxalate (hereinafter, these are also collectively referred to as "oxalic acid (salt)") are difficult to be oxidized, it is difficult to extract electrons from the oxalic acid (salt) with a photocatalyst in the related art. In addition, even when electrons can be extracted, it is difficult to transfer the extracted electrons to the organic compound. Therefore, in a reduction reaction using the photocatalyst in the related art, the oxalic acid (salt) is unsuitable as the reducing agent.

However, since the polycyclic compound according to the embodiment of the present invention has high oxidizing power and high reducing power, it can easily extract electrons from the oxalic acid (salt) and can further easily transfer the extracted electrons to the organic compound. Therefore, according to the present invention, the oxalic acid (salt) can be suitably used as the reducing agent.

Since the oxalic acid (salt) becomes carbon dioxide when oxidized, it hardly inhibits the reduction reaction even when it remains in the reaction system. Moreover, since the oxalic acid (salt) is soluble in water and available at low cost, the reduction reaction can be carried out in water at low cost.

A proportion of the reducing agent is preferably 1 to 10 equivalents, more preferably 1 to 6 equivalents, and still more preferably 2 to 4 equivalents with respect to the organic compound.

The reduction reaction is carried out in a solvent.

Examples of the solvent include water; dimethyl sulfoxide; nitrile-based solvents such as acetonitrile and benzonitrile; alcohol-based solvents such as methanol, ethanol, and propanol; saturated aliphatic hydrocarbon-based solvents such as hexane; ether-based solvents such as tetrahydrofuran, diethyl ether, cyclopentyl methyl ether, and methyl t-butyl ether; aromatic hydrocarbon-based solvents such as toluene and xylene; and amide-based solvents such as dimethylformamide and dimethylacetamide.

The solvent may be used alone or in combination of two or more kinds at an arbitrary ratio.

Reaction conditions can be appropriately set depending on chemical properties and/or physical properties of the substrate.

A reaction temperature of the reduction reaction is preferably 0°C to 100°C, and more preferably 25°C to 50°C.

A reaction time of the reduction reaction is preferably 1 to 48 hours, and more preferably 2 to 24 hours.

Since the polycyclic compound according to the embodiment of the present invention is used as a photocatalyst in the method for reducing an organic compound according to the embodiment of the present invention described above, visible light can be used as an energy source, and the reduction reaction can be carried out under mild conditions.

Moreover, according to the present invention, oxalic acid (salt) can be suitably used as a reducing agent. In addition, since the polycyclic compound according to the embodiment of the present invention is soluble in water as well as in an organic solvent, water can be used as a reaction solvent.

### [Coupling method]

A method for coupling organic compounds according to an embodiment of the present invention (hereinafter, also referred to as "coupling reaction") is a method of coupling organic compounds (hereinafter, one is also referred to as "organic compound (I)" and the other is also referred to as "organic compound (II)") in the presence of the polycyclic compound according to the embodiment of the present invention described above.

Examples of the organic compound (I) and the organic compound (II) suitable for the coupling method according to the embodiment of the present invention include a carbonyl compound, an imine compound, a halide, a nitrile compound, a nitro compound, a hydroxy compound, and an unsaturated aliphatic hydrocarbon.

The organic compound (I) and the organic compound (II) may be the same compound or different compounds. That is, the coupling reaction of the organic compounds in the present invention may be a homo-coupling reaction or a cross-coupling reaction.

At least one of the organic compound (I) and the organic compound (II) is preferably a carbonyl compound, and particularly preferably an ester compound. That is, the coupling method according to the embodiment of the present invention is suitable as a coupling method of coupling a carbonyl compound and an organic compound, particularly as a coupling method of coupling an ester compound and an organic compound.

Preferable combinations of the organic compound (I) and the organic compound (II) include, for example, a combination of carbonyl compounds, a combination of a carbonyl compound and a halide, a combination of a carbonyl compound and an unsaturated aliphatic hydrocarbon, and a combination of halides.

Examples of the coupling reaction include the following (i) to (iv).
(i): Reductive coupling reaction between an ester compound represented by Formula (12) and a carbonyl compound represented by Formula (13) 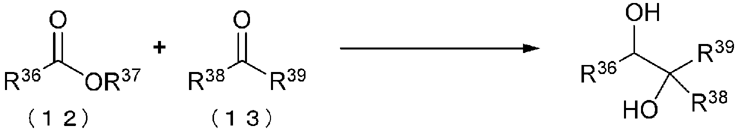
(ii): Coupling reaction between an ester compound represented by Formula (12) and a halide represented by Formula (14) 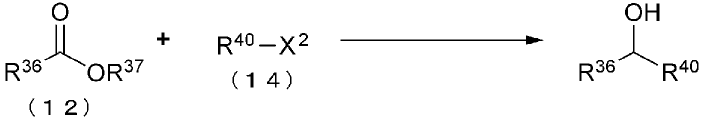
(iii): Coupling reaction between an ester compound represented by Formula (12) and an alkene represented by Formula (15) 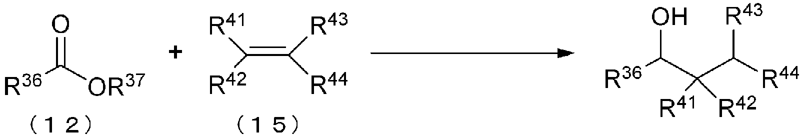
(iv): Coupling reaction between halides represented by Formula (16) and Formula (17)

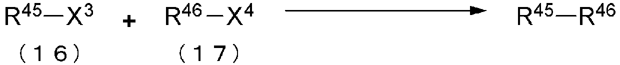

In the formulae of the coupling reactions shown above, R³⁶ and R³⁷ are each independently a hydrocarbon group, an alkoxy group, an amino group, a monoalkylamino group, a dialkylamino group, a hydroxy group, or a hydrogen atom.

R³⁸ and R³⁹ are each independently a hydrocarbon group, an alkoxy group, an amino group, a monoalkylamino group, a dialkylamino group, a hydroxy group, or a hydrogen atom.

R⁴⁰ is a hydrocarbon group, and X² is a bromine atom, a chlorine atom, or an iodine atom.

R⁴¹ to R⁴⁴ are each independently a hydrocarbon group, an alkoxy group, an amino group, or a hydrogen atom.

R⁴⁵ and R⁴⁶ are each independently a hydrocarbon group, and X³ and X⁴ are each independently a bromine atom, a chlorine atom, or an iodine atom. In particular, R⁴⁵ and R⁴⁶ are each independently preferably an aryl group.

Here, the hydrocarbon group, the alkoxy group, the monoalkylamino group, and the dialkylamino group include the hydrocarbon groups, alkoxy groups, monoalkylamino groups, and dialkylamino groups mentioned above in the description of the polycyclic compound.

In the coupling method according to the embodiment of the present invention, the total concentration of the organic compound (I) and the organic compound (II) in 100% by mass of a reaction solution is preferably 0.002% to 20% by mass, more preferably 0.002% to 2% by mass, and still more preferably 0.02% to 0.2% by mass.

A reaction proportion of the organic compound (I) and the organic compound (II) is such that an amount of the organic compound (II) is preferably 1 to 100 equivalents, more preferably 1 to 50 equivalents, and still more preferably 1 to 10 equivalents with respect to the organic compound (I).

When the coupling reaction is, for example, (i) to (iii) described above, the ester compound may be the organic compound (I) or the organic compound (II), but is preferably the organic compound (I).

A proportion of the polycyclic compound is preferably 0.001 to 0.2 equivalents, more preferably 0.005 to 0.1 equivalents, and still more preferably 0.01 to 0.05 equivalents with respect to the organic compound (1).

As described above, since the polycyclic compound according to the embodiment of the present invention can absorb ultraviolet-visible light and can use the absorbed light as an energy source, the coupling reaction of the organic compound is preferably carried out while irradiating ultraviolet rays or visible light, and more preferably carried out while irradiating visible light.

Visible light and irradiation amount and output thereof are the same as in the reduction reaction of the organic compound described above.

The coupling reaction is usually carried out in the presence of a reducing agent.

Examples of the reducing agent include those mentioned above in the description of the reduction reaction of the organic compound.

As described above, according to the present invention, oxalic acid (salt) can be suitably used as the reducing agent.

A proportion of the reducing agent is preferably 1 to 20 equivalents, more preferably 1 to 12 equivalents, and still more preferably 2 to 8 equivalents with respect to the organic compound (I).

The coupling reaction is carried out in a solvent.

Examples of the solvent include those mentioned above in the description of the reduction reaction of the organic compound.

Reaction conditions can be appropriately set depending on chemical properties and/or physical properties of the substrate.

A reaction temperature of the coupling reaction is preferably 0°C to 100°C, and more preferably 25°C to 50°C.

A reaction time of the coupling reaction is preferably 1 to 48 hours, and more preferably 12 to 24 hours.

Since the polycyclic compound according to the embodiment of the present invention is used as a photocatalyst in the method for coupling organic compounds according to the embodiment of the present invention described above, visible light can be used as an energy source, and the coupling reaction can be carried out under mild conditions.

Moreover, according to the present invention, oxalic acid (salt) can be suitably used as a reducing agent. In addition, since the polycyclic compound according to the embodiment of the present invention is soluble in water as well as in an organic solvent, water can be used as a reaction solvent.

### Examples

Hereinafter, the present invention will be described in detail with Examples, but the present invention is not limited to the following description.

### Example A1: Synthesis of compound (1a-1)

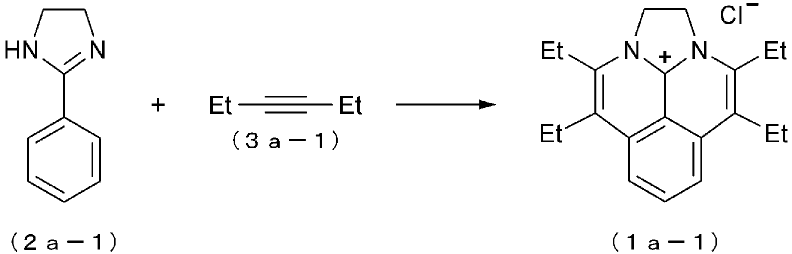

589 mg of a compound (2a-1) and 645 mg of a compound (3a-1) were charged into a reaction vessel, 20 mL of methanol was added to dissolve them, and then 62 mg of [Rh(Cp*)Cl₂]₂ and 2790 mg of silver acetate were added thereto. Next, the inside of the reaction vessel was replaced with nitrogen, and then the mixture was stirred at 80°C for 16 hours under a nitrogen atmosphere. After cooling, a saturated sodium chloride aqueous solution and dichloromethane were added thereto, the target substance was extracted from an aqueous layer with dichloromethane, and the obtained organic layers were combined and dried to collect a solid. The collected solid was purified by column chromatography and dried to obtain a compound (1a-1) as a yellow solid with an amount of 620 mg and a yield of 45%.

Physicochemical data of the obtained compound (1a-1) are shown below.

¹H-NMR (400 MHz, solvent: CDCl₃): δ (ppm) = 7.87 (t, J = 8.0 Hz, 1H), 7.36 (d, J = 8.4 Hz, 2H), 5.23 (s, 4H), 2.82 (q, J = 8.0 Hz, 4H), 2.72 (q, J = 8.0 Hz, 4H), 1.34 (t, J = 8.0 Hz, 6H), 1.21 (t, J = 8.0 Hz, 6H).

¹³C-NMR (101 MHz, solvent: CDCl₃): δ (ppm) = 148.1, 140.1, 137.3, 136.5, 121.3, 117.0, 116.5, 48.8, 22.1, 20.2, 13.5, 13.1.

HRMS (ESI+): Calcd for C₂₁H₂₇N₂; [M-Cl]⁺ 307.2169; Found m/z 307.2177.

IR (ATR): 2973 (m), 1665 (s), 1603 (s), 1580 (s), 1168 (s) cm⁻¹.

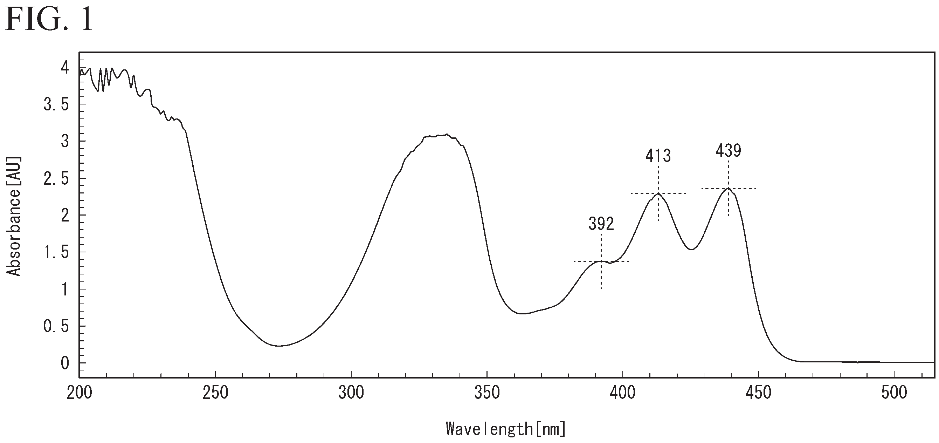

An ultraviolet-visible absorption spectrum of the compound (1a-1) was measured using an ultraviolet-visible spectrophotometer (manufactured by Agilent Technologies, Inc., product name "Agilent 8453"). The results are shown in FIG. 1.

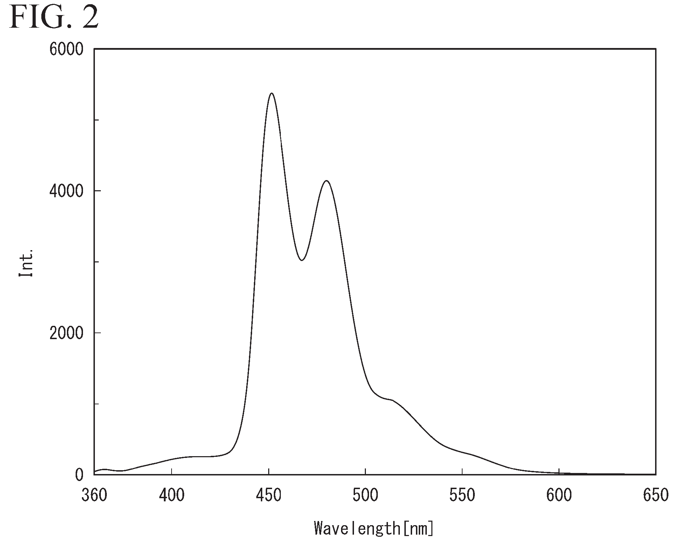

In addition, a fluorescence spectrum of the compound (1a-1) was measured at an excitation wavelength of 350 nm using a fluorescence spectrophotometer (manufactured by JASCO Corporation, product name "FP-8350"). The results are shown in FIG. 2.

### Example A2: Synthesis of compound (1a-2)

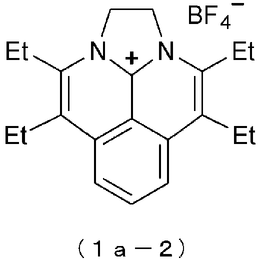

10.0 mg of the compound (1a-1) obtained in Example A1 and 8.5 mg of silver(1) tetrafluoroborate (AgBF₄) were charged into a reaction vessel, the inside of the reaction vessel was replaced with nitrogen, 0.9 mL of dichloromethane was added to dissolve them, and then the mixture was stirred at room temperature for 2 hours. Next, the reaction product was filtered, and the filtrate was dried to obtain a compound (1a-2) as a yellow solid with an amount of 9.8 mg and a yield of 85%.

Physicochemical data of the obtained compound (1a-2) are shown below.

¹H-NMR (400 MHz, solvent: CDCl₃): δ (ppm) = 7.88 (t, J = 8.2 Hz, 1H), 7.37 (d, J = 7.8 Hz, 2H), 5.02 (s, 4H), 2.79 (q, J = 7.5 Hz, 4H), 2.73 (q, J = 7.3 Hz, 4H), 1.33 (t, J = 7.8 Hz, 6H), 1.22 (t, J = 7.5 Hz, 6H).

¹³C-NMR (101 MHz, solvent: CDCl₃): δ (ppm) = 148.4, 140.1, 137.5, 136.7, 121.6, 117.3, 116.6, 48.4, 22.2, 20.4, 13.5, 13.3.

¹⁹F-NMR (373 MHz, solvent: CDCl₃): δ (ppm) = -155.1 (trifluoromethylbenzene was used as an internal standard; δ = -63.7).

HRMS (ESI+): Calcd for C₂₁H₂₇N₂; ([M]⁺) 307.2169; Found m/z 307.2175.

IR (ATR): 2973 (m), 1666 (s), 1604 (s), 1581 (s), 1052 (s) cm⁻¹.

### Example A3: Synthesis of compound (1a-3)

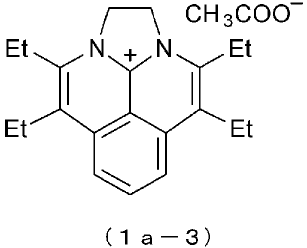

9.9 mg of the compound (1a-1) obtained in Example A1 and 7.5 mg of silver acetate (CH₃COOAg) were charged into a reaction vessel, the inside of the reaction vessel was replaced with nitrogen, 0.9 mL of dichloromethane was added to dissolve them, and then the mixture was stirred at room temperature for 2 hours. Next, the reaction product was filtered, and the filtrate was dried to obtain a compound (1a-3) as a brown solid with an amount of 10.2 mg and a yield of 96%.

Physicochemical data of the obtained compound (1a-3) are shown below.

¹H-NMR (400 MHz, solvent: methanol-d4): δ (ppm) = 7.97 (t, J = 8.2 Hz, 1H), 7.53 (d, J = 8.2 Hz, 2H), 4.79 (s, 4H), 2.84 (q, J = 7.6 Hz, 4H), 2.83 (q, J = 7.5 Hz, 4H), 1.89 (s, 3H), 1.34 (t, J = 7.5 Hz, 6H), 1.25 (t, J = 7.5 Hz, 6H).

¹³C-NMR (101 MHz, solvent: methanol-d4): δ (ppm) = 180.1, 150.1, 141.4, 138.6, 137.8, 122.4, 118.4, 117.8, 49.1, 24.1, 22.7, 21.0, 13.6, 13.5.

HRMS (ESI+): Calcd for C₂₁H₂₇N₂; ([M]⁺) 307.2169; Found m/z 307.2178.

IR (ATR): 2968 (m), 1667 (s), 1605 (s), 1580 (s) cm⁻¹.

### Example A4: Synthesis of compound (1a-4)

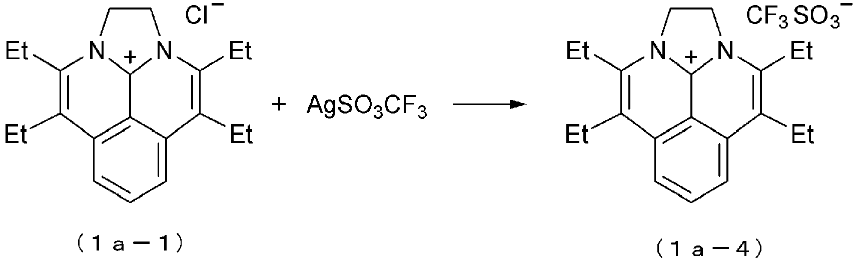

34.5 mg of the compound (1a-1) obtained in Example A1 and 25.5 mg of silver trifluoromethanesulfonate (AgSO₃CF₃) were charged into a reaction vessel, the inside of the reaction vessel was replaced with nitrogen, 1.3 mL of dichloromethane was added to dissolve them, and then the mixture was stirred at room temperature for 4 hours. Next, the reaction product was filtered, and the filtrate was dried to obtain a compound (1a-4) as a yellow solid with an amount of 45.7 mg and a yield of 99%.

Physicochemical data of the obtained compound (1a-4) are shown below.

¹H-NMR (400 MHz, solvent: CDCl₃): δ (ppm) = 7.88 (t, J = 8.0 Hz, 1H), 7.38 (d, J = 8.0 Hz, 2H), 4.95 (s, 4H), 2.77 (q, J = 7.6 Hz, 4H), 2.73 (q, J = 8.0 Hz, 4H), 1.32 (t, J = 8.0 Hz, 6H), 1.22 (t, J = 8.0 Hz, 6H).

¹³C-NMR (101 MHz, solvent: CDCl₃): δ (ppm) = 148.2, 139.9, 137.3, 136.6, 121.5, 117.2, 116.4, 48.2, 22.0, 20.2, 13.3, 13.1.

¹⁹F-NMR (373 MHz, solvent: CDCl₃): δ (ppm) = 79.4 (trifluoromethylbenzene was used as an internal standard; δ = -63.7).

HRMS (ESI+): Calcd for C₂₁H₂₇N₂; [M-OTf]⁺ 307.2169; Found m/z 307.2173.

IR (ATR): 2979 (m), 1667 (s), 1606 (s), 1583 (s), 1262 (s) cm⁻¹.

### Example A5: Synthesis of compound (1a-5)

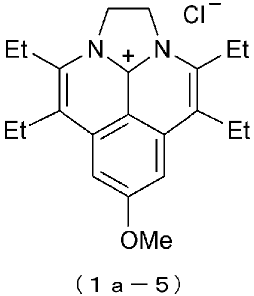

A compound (1a-5) was obtained as a yellow solid with an amount of 50 mg and a yield of 5% in the same manner as in Example A1, except that the compound (2a-1) was changed to 2-(4-methoxyphenyl)-2-imidazoline.

Physicochemical data of the obtained compound (1a-5) are shown below.

¹H-NMR (400 MHz, solvent: CDCl₃): δ (ppm) = 6.81 (s, 2H), 5.16 (s, 4H), 4.00 (s, 3H), 2.80 (q, J = 7.7 Hz, 4H), 2.68 (q, J = 7.6 Hz, 4H), 1.32 (t, J = 7.5 Hz, 6H), 1.21 (t, J = 7.7 Hz, 6H).

¹³C-NMR (101 MHz, solvent: CDCl₃): δ (ppm) = 166.6, 147.0, 140.7, 139.2, 120.4, 111.4, 102.9, 55.8, 48.6, 22.2, 20.2, 13.5, 12.9.

HRMS (ESI+): Calcd for C₂₂H₂₉N₂; ([M]⁺) 337.22744; Found m/z 337.22822.

IR (ATR): 2969 (M), 1667 (S), 1602 (S), 1577 (S), 1397 (s), 1216 (s), 1047 (s) cm⁻¹.

### Example A6: Synthesis of compound (1a-6)

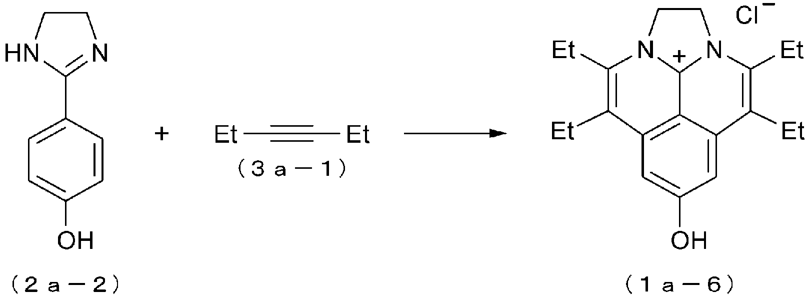

975 mg of a compound (2a-2) and 994 mg of a compound (3a-1) were charged into a reaction vessel, 30 mL of methanol was added to dissolve them, and then 92 mg of [Rh(Cp*)Cl₂]₂ and 4210 mg of silver acetate were added thereto. Next, the inside of the reaction vessel was replaced with nitrogen, and then the mixture was stirred at 80°C for 20 hours under a nitrogen atmosphere. After cooling, a saturated sodium chloride aqueous solution and dichloromethane were added thereto, the target substance was extracted from an aqueous layer with dichloromethane, the obtained organic layers were combined and dried to collect a solid, and the collected solid was purified by column chromatography. Thereafter, reprecipitation was carried out using acetonitrile and toluene, and the precipitate substance was collected and dried to obtain a compound (1a-6) as a yellow solid with an amount of 127 mg and a yield of 6%.

Physicochemical data of the obtained compound (1a-6) are shown below.

¹H-NMR (400 MHz, solvent: CD₃OD): δ (ppm) = 6.85 (s, 2H), 4.70 (s, 4H), 2.79 (q, J = 7.3 Hz, 4H), 2.73 (q, J = 7.8 Hz, 4H), 1.31 (t, J = 7.8 Hz, 6H), 1.22 (t, J = 7.8 Hz, 6H).

¹³C-NMR (101 MHz, solvent: CD₃OD): δ (ppm) = 167.0, 148.7, 141.6, 140.8, 121.2, 111.7, 105.7, 22.7, 21.1, 13.5, 13.4.

HRMS (ESI+): Calcd for C₂₁H₂₇N₂O; [M-Cl]⁺ 323.2118; Found m/z 323.2122.

IR (ATR): 3579 (br), 3436 (br), 3200 to 2400 (br), 1668 (s), 1574 (vs), 1409 (s) cm⁻¹.

### Example A7: Synthesis of compound (1a-7)

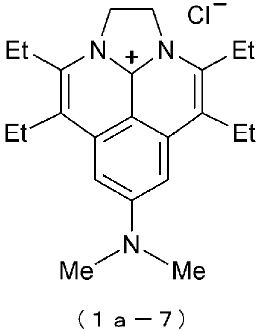

A compound (1a-7) was obtained as an ocher solid with an amount of 33 mg and a yield of 4% in the same manner as in Example 1A, except that the compound (2a-1) was changed to 2-(4-dimethylaminophenyl)-2-imidazoline.

Physicochemical data of the obtained compound (1a-7) are shown below.

¹H-NMR (400 MHz, solvent: CDCl₃): δ (ppm) = 6.47 (s, 2H), 5.05 (s, 4H), 3.20 (s, 6H), 2.75 (q, J = 7.6 Hz, 4H), 2.65 (q, J = 7.5 Hz, 4H), 1.30 (t, J = 7.6 Hz, 6H), 1.20 (t, J = 7.6 Hz, 6H).

¹³C-NMR (101 MHz, solvent: CDCl₃): δ (ppm) = 155.7, 146.4, 139.9, 137.9, 119.5, 107.7, 99.8, 48.2, 40.5, 22.1, 20.1, 13.5, 12.8.

HRMS (ESI+): Calcd for C₂₂H₂₉N₂; ([M]⁺) 350.25907; Found m/z 350.25800.

IR (ATR): 2969 (m), 1668 (s), 1579 (s), 1420 (s) cm⁻¹.

### Example A8: Synthesis of compound (1a-8)

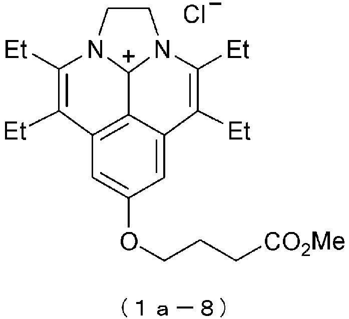

A compound (1a-8) was obtained as a pale yellow solid with an amount of 94 mg and a yield of 34% in the same manner as in Example 1A, except that the compound (2a-1) was changed to 2-(4-(3-methoxycarbonylpropyloxy)phenyl)-2-imidazoline.

Physicochemical data of the obtained compound (1a-8) are shown below.

¹H-NMR (400 MHz, solvent: CDCl₃): δ (ppm) = 6.79 (s, 2H), 5.18 (s, 4H), 4.21 (t, J = 6.2 Hz, 2H), 3.73 (s, 3H), 2.79 (q, J = 7.6 Hz, 4H), 2.67 (q, J = 7.5 Hz, 4H), 2.60 (t, J = 7.1 Hz, 2H), 2.21 (tt, J = 6.9, 6.9 Hz, 2H), 1.32 (t, J = 7.8 Hz, 6H), 1.20 (t, J = 7.6 Hz, 6H).

¹³C-NMR (101 MHz, solvent: CDCl₃): δ (ppm) = 173.4, 165.8, 147.0, 140.6, 139.2, 120.4, 111.4, 103.3, 67.3, 51.7, 48.6, 30.1, 24.3, 22.2, 20.2, 13.4, 12.9.

HRMS (ESI+): Calcd for C₂₆H₃₅N₂O₃; ([M]⁺) 423.26422; Found m/z 423.26377.

IR (ATR): 2968 (m), 1723 (s), 1670 (s), 1201 (s) cm⁻¹.

### Example A9: Synthesis of compound (1a-9)

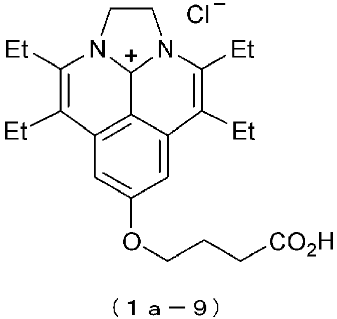

2 mL of water and 19 mg of lithium hydroxide (LiOH) were added to 39 mg of the compound (1a-8) obtained in Example A8, and the mixture was stirred at 50°C for 25 hours and then concentrated under reduced pressure to obtain a solid. The obtained solid was purified by column chromatography to obtain a compound (1a-9) as a pale yellow solid with an amount of 35 mg and a yield of 93%.

Physicochemical data of the obtained compound (1a-9) are shown below.

¹H-NMR (400 MHz, solvent: methanol-d4): δ (ppm) = 6.96 (s, 2H), 4.75 (s, 4H), 4.26 (t, J = 6.4 Hz, 2H), 2.83 to 2.74 (m, 8H), 2.51 (t, J = 7.4 Hz, 2H), 2.14 (tt, J = 6.9, 6.9 Hz, 2H), 1.32 (t, J = 7.6 Hz, 6H), 1.23 (t, J = 7.6 Hz, 6H).

¹³C-NMR (101 MHz, solvent: methanol-d4): δ (ppm) = 177.7, 167.5, 148.8, 142.0, 140.6, 121.6, 112.5, 104.6, 69.0, 48.9, 31.8, 25.8, 22.8, 21.0, 13.5, 13.4.

HRMS (ESI+): Calcd for C₂₅H₃₃N₂O₃; ([M]⁺) 409.24857; Found m/z 409.24730.

IR (ATR): 3540 to 2600 (br), 2925 (m), 1667 (s), 1577 (s), 1173 (s) cm⁻¹.

### Example A10: Synthesis of compound (1a-10)

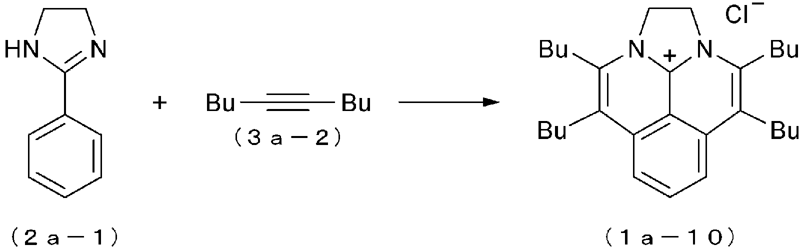

176 mg of the compound (2a-1) and 336 mg of the compound (3a-2) were charged into a reaction vessel, 6 mL of methanol was added to dissolve them, and then 18 mg of [Rh(Cp*)Cl₂]₂ and 842 mg of silver acetate were added thereto. Next, the inside of the reaction vessel was replaced with nitrogen, and then the mixture was stirred at 80°C for 16 hours under a nitrogen atmosphere. After cooling, a saturated sodium chloride aqueous solution and dichloromethane were added thereto, the target substance was extracted from an aqueous layer with dichloromethane, and the obtained organic layers were combined and dried to collect a solid. The collected solid was purified by column chromatography and dried to obtain a compound (1a-10) as a yellow solid with an amount of 112 mg and a yield of 21%.

Physicochemical data of the obtained compound (1a-10) are shown below.

¹H-NMR (396 MHz, solvent: CDCl₃): δ (ppm) = 7.85 (t, J = 8.2 Hz, 1H), 7.32 (d, J = 8.2 Hz, 2H), 5.20 (s, 4H), 2.75 (t, J = 8.6 Hz, 4H), 2.65 (t, J = 8.2 Hz, 4H), 1.67 to 1.49 (m, 16H), 1.02 to 0.98 (m, 12H).

¹³C-NMR (101 MHz, solvent: CDCl₃): δ (ppm) = 148.2, 139.4, 137.6, 136.6, 120.5, 117.2, 116.5, 49.1, 31.3, 30.7, 28.7, 27.0, 23.1, 22.9, 14.0, 13.9.

HRMS (ESI+): Calcd for C₂₉H₄₃N₂; [M-Cl]⁺ 419.3421; Found m/z 419.3430.

IR (ATR): 2957 (m), 2870 (m), 1667 (m), 1604 (s), 1581 (m) cm⁻¹.

### Example A11: Synthesis of compound (1a-11)

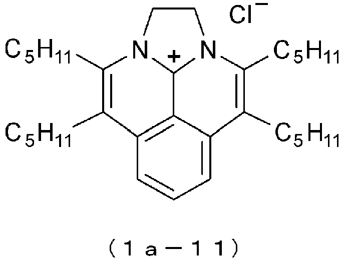

C₅H₁₁ in the formula represents n-pentyl.

A compound (1a-11) was obtained as a yellow solid with an amount of 334 mg and a yield of 15% in the same manner as in Example 1A, except that the compound (3a-1) was changed to dodeca-6-yne.

Physicochemical data of the obtained compound (1a-11) are shown below.

¹H-NMR (400 MHz, solvent: CDCl₃): δ (ppm) = 7.83 (t, J = 8.0 Hz, 1H), 7.30 (d, J = 8.0 Hz, 2H), 5.17 (s, 4H), 2.73 (t, J = 8.0 Hz, 4H), 2.63 (t, J = 7.6 Hz, 4H), 1.66 to 1.34 (m, 24H), 0.93 (t, J = 7.2 Hz, 12H).

¹³C-NMR (101 MHz, solvent: CDCl₃): δ (ppm) = 148.2, 139.5, 137.6, 136.6, 120.5, 117.2, 116.5, 49.1, 32.2, 31.9, 29.1, 29.0, 28.4, 27.3, 22.6, 22.5, 14.2, 14.1.

HRMS (ESI+): Calcd for C₃₃H₅₁N₂; ([M]⁺) 475.4047; Found m/z 475.4048.

IR (ATR): 2955 (m), 1667 (s), 1580 (m) cm⁻¹.

### Example A12: Synthesis of compound (1b-1)

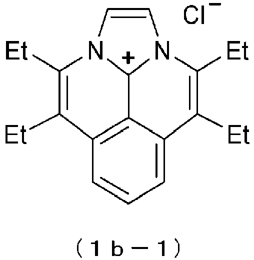

A compound (1b-1) was obtained as a yellow solid with an amount of 85 mg and a yield of 7% in the same manner as in Example 1A, except that the compound (2a-1) was changed to 2-phenyl-2-imidazole.

Physicochemical data of the obtained compound (1b-1) are shown below.

¹H-NMR (400 MHz, solvent: CDCl₃): δ (ppm) = 9.47 (s, 2H), 8.30 (dd, J = 8.2, 7.8 Hz, 1H), 8.16 (d, J = 8.2 Hz, 2H), 3.49 (q, J = 7.6 Hz, 4H), 3.20 (q, J = 7.6 Hz, 4H), 1.51 (t, J = 7.6 Hz, 6H), 1.41 (t, J = 7.6 Hz, 6H).

¹³C-NMR (101 MHz, solvent: CDCl₃): δ (ppm) = 136.1, 133.1, 131.7, 130.7, 126.5, 120.4, 118.2, 113.2, 22.1, 21.0, 14.2, 12.7.

HRMS (ESI+): Calcd for C₂₁H₂₅N₂; ([M]⁺) 305.20123; Found m/z 305.20184.

IR (ATR): 2970 (m), 1686 (m), 1569 (s), 1052 (s) cm⁻¹.

### Comparative Example A1: Synthesis of compound (C-1)

A compound (C-1) of the following formula was synthesized by the method described in Org. Lett., 2017, Vol. 19, No. 9, p. 1702.

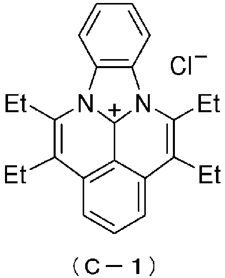

### Comparative Example A2: Synthesis of compound (C-2)

A compound (C-2) of the following formula was synthesized by the method described in Org. Biomol. Chem., 2015, Vol. 13, No. 2, p. 447.

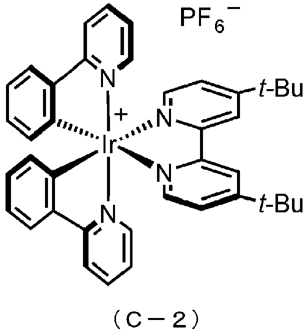

### Comparative Example A3: Compound (C-3)

As a compound (C-3) of the following formula, a product name "[Ru(bpz)₃][PF₆]₂" manufactured by Aldrich was used.

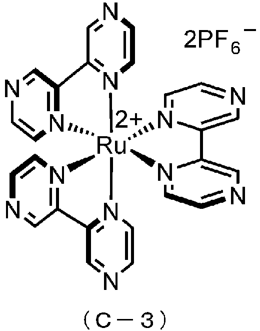

### Comparative Example A4: Synthesis of compound (C-4)

A compound (C-4) of the following formula was synthesized by the method described in Chem. Sci., 2018, Vol. 9, No. 36, p. 7230.

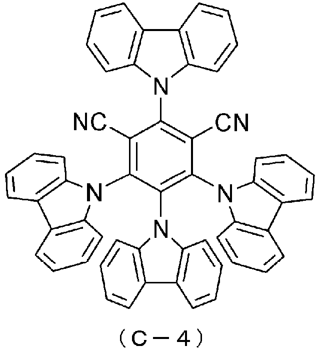

### Example 1

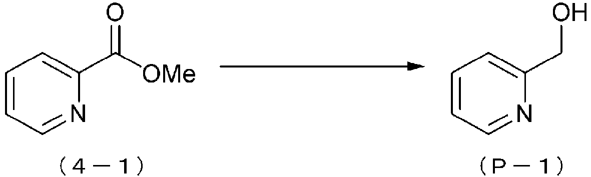

27.7 mg of a compound (4-1) as an ester compound, 2.10 mg of the compound (1a-1) as a photocatalyst, and 114 mg of ammonium oxalate as a reducing agent were charged into a reaction vessel, 1.6 mL of water and 1.6 mL of acetonitrile were added thereto, and then the mixture was stirred at 40°C for 12 hours while irradiating with blue light (manufactured by Kessil, product name "A160WE Tuna Blue", output 40 W, wavelength 462 nm). After cooling, acetone was added thereto, the solid was removed by filtration, and the filtrate was dried to obtain a compound (P-1).

The yield of the obtained compound (P-1) was determined by GC measurement. The results are shown in Table 1.

The GC measurement is shown below.

### (GC measurement)

· Device: manufactured by Agilent Technologies, Inc., product name "Agilent 6850" · Column: manufactured by Agilent Technologies, Inc., product name "HP-5MS"
· Column length: 30 m
· Injection volume: 1 µL
· Inlet temperature: 250°C
· Column oven temperature: held at 40°C for 1 minute, and then heated from 40°C to 300°C at 30 °C/min; thereafter, held at 300°C for 15 minutes.
· Carrier gas: helium

### Example 2

The ester compound was reduced to obtain the compound (P-1) in the same manner as in Example 1, except that 2.71 mg of the compound (1a-4) was used as a photocatalyst.

The yield is shown in Table 1.

### Example 2a

The ester compound was reduced to obtain the compound (P-1) with a yield of 59% in the same manner as in Example 1, except that 2.2 mg of the compound (1a-5) was used as a photocatalyst.

### Comparative Example 1

An attempt was made to reduce the ester compound in the same manner as in Example 1, except that 2.08 mg of the compound (C-1) was used as a photocatalyst, but the reduction could not be carried out, and the compound (P-1) was not obtained.

### Comparative Example 2

The ester compound was reduced to obtain the compound (P-1) in the same manner as in Example 1, except that 5.50 mg of the compound (C-2) was used as a photocatalyst.

The yield is shown in Table 1.

### Comparative Example 3

An attempt was made to reduce the ester compound in the same manner as in Example 1, except that 5.11 mg of the compound (C-3) was used as a photocatalyst, but the reduction could not be carried out, and the compound (P-1) was not obtained.

### Comparative Example 4

An attempt was made to reduce the ester compound in the same manner as in Example 1, except that 4.67 mg of the compound (C-4) was used as a photocatalyst, but the reduction could not be carried out, and the compound (P-1) was not obtained.

**[Table 1]**

| | Organic compound | Photocatalyst | Yield [%] |
|---|---|---|---|
| Example 1 | Compound (4-1) | Compound (1a-1) | 89 |
| Example 2 | Compound (4-1) | Compound (1a-4) | 69 |
| Comparative Example 1 | Compound (4-1) | Compound (C-1) | 0 |
| Comparative Example 2 | Compound (4-1) | Compound (C-2) | 2 |
| Comparative Example 3 | Compound (4-1) | Compound (C-3) | 0 |
| Comparative Example 4 | Compound (4-1) | Compound (C-4) | 0 |

As is clear from Table 1 and the result of Example 2a, with the polycyclic compound according to the embodiment of the present invention, visible light could be used as an energy source to reduce an ester compound to an alcohol with a high yield.

On the other hand, the compound (C-1) used in Comparative Example 1 did not have a function of a photocatalyst using visible light as an energy source, and could not reduce the ester compound.

Although the compounds (C-2) to (C-4) used in Comparative Examples 2 to 4 were general photocatalysts, they could not reduce the ester compound, or even when they could partially reduce the ester compound, the results were very poor in yield.

### Example 3

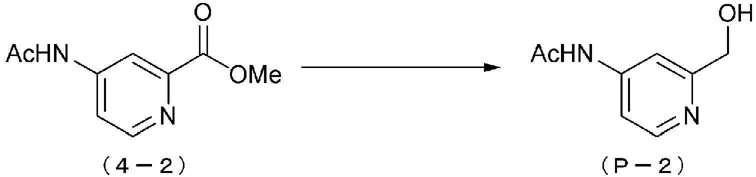

38.2 mg of a compound (4-2) as an ester compound, 2.04 mg of the compound (1a-1) as a photocatalyst, and 62.7 mg of ammonium oxalate as a reducing agent were charged into a reaction vessel, 1.6 mL of water and 1.6 mL of acetonitrile were added thereto, and then the mixture was stirred at 40°C for 3 hours while irradiating with blue light (manufactured by Kessil, product name "A160WE Tuna Blue", output 40 W, wavelength 462 nm). After cooling, acetone was added thereto, the solid was removed by filtration, and the filtrate was purified by column chromatography and dried to obtain the following compound (P-2).

The yield of the obtained compound (P-2) was determined by NMR measurement. The results are shown in Table 2.

### Example 4

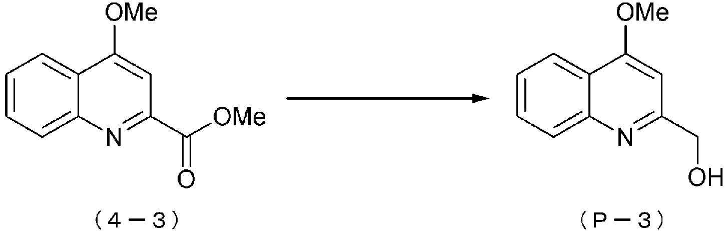

43.4 mg of a compound (4-3) as an ester compound, 2.09 mg of the compound (1a-1) as a photocatalyst, and 114 mg of ammonium oxalate as a reducing agent were charged into a reaction vessel, 1.6 mL of water and 1.6 mL of acetonitrile were added thereto, and then the mixture was stirred at 50°C for 8 hours while irradiating with blue light (manufactured by Kessil, product name "A160WE Tuna Blue", output 40 W, wavelength 462 nm). After cooling, a saturated sodium chloride aqueous solution and dichloromethane were added thereto, the organic layer was extracted, and the extracted organic layer was purified by column chromatography and dried to obtain the following compound (P-3).

The yield of the obtained compound (P-3) was determined by NMR measurement. The results are shown in Table 2.

### Example 5

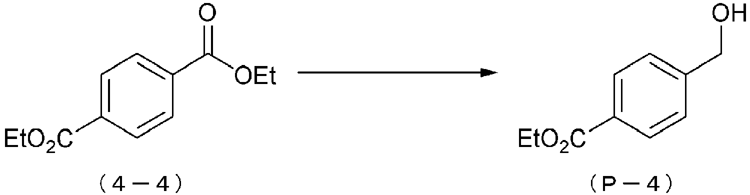

44.3 mg of a compound (4-4) as an ester compound, 2.11 mg of the compound (1a-1) as a photocatalyst, and 113 mg of ammonium oxalate as a reducing agent were charged into a reaction vessel, 1.6 mL of water and 1.6 mL of acetonitrile were added thereto, and then the mixture was stirred at 50°C for 12 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-4).

The yield of the obtained compound (P-4) was determined by NMR measurement. The results are shown in Table 2.

### Example 6

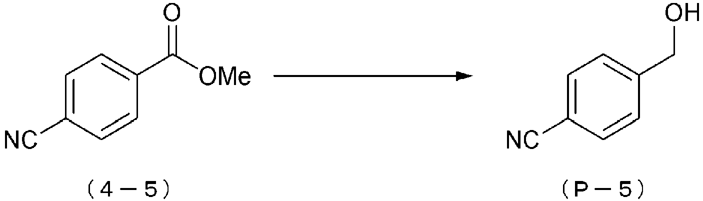

32.2 mg of a compound (4-5) as an ester compound, 2.04 mg of the compound (1a-1) as a photocatalyst, and 114 mg of ammonium oxalate as a reducing agent were charged into a reaction vessel, 1.6 mL of water and 1.6 mL of acetonitrile were added thereto, and then the mixture was stirred at 50°C for 12 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-5).

The yield of the obtained compound (P-5) was determined by NMR measurement. The results are shown in Table 2.

### Example 7

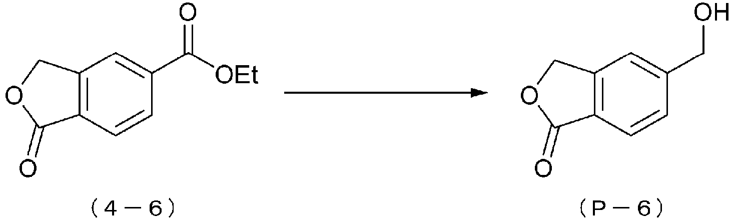

39.0 mg of a compound (4-6) as an ester compound, 2.07 mg of the compound (1a-1) as a photocatalyst, and 62.5 mg of ammonium oxalate as a reducing agent were charged into a reaction vessel, 1.6 mL of water and 1.6 mL of acetonitrile were added thereto, and then the mixture was stirred at 40°C for 2 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-6).

The yield of the obtained compound (P-6) was determined by NMR measurement. The results are shown in Table 2.

**[Table 2]**

| | Organic compound | Photocatalyst | Yield [%] |
|---|---|---|---|
| Example 3 | Compound (4-2) | Compound (1a-1) | 76 |
| Example 4 | Compound (4-3) | Compound (1a-1) | 52 |
| Example 5 | Compound (4-4) | Compound (1a-1) | 89 |
| Example 6 | Compound (4-5) | Compound (1a-1) | 77 |
| Example 7 | Compound (4-6) | Compound (1a-1) | 65 |

As is clear from Table 2, with the polycyclic compound according to the embodiment of the present invention, visible light could be used as an energy source to reduce an ester compound to an alcohol with a high yield.

### Example 8

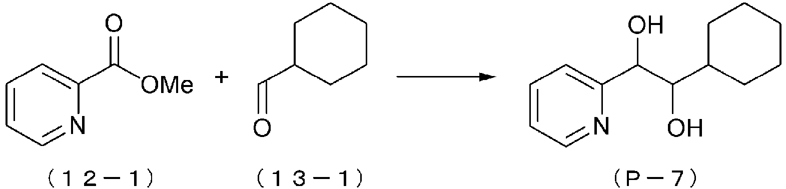

28.1 mg of a compound (12-1) as an ester compound (organic compound (I)), 112 mg of a compound (13-1) as a carbonyl compound (organic compound (11)), 3.40 mg of the compound (1a-1) as a photocatalyst, and 84.6 mg of ammonium oxalate as a reducing agent were charged into a reaction vessel, 1.6 mL of water and 1.6 mL of acetonitrile were added thereto, and then the mixture was stirred at 40°C for 24 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-7).

The yield of the obtained compound (P-7) was determined by NMR measurement. The results are shown in Table 3.

### Example 9

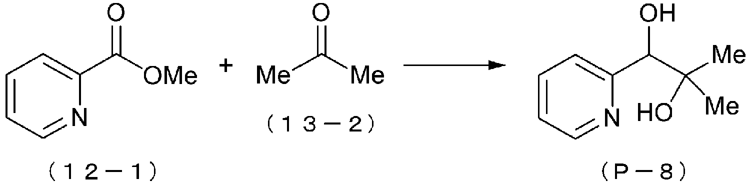

28.3 mg of the compound (12-1) as an ester compound (organic compound (I)), 3.41 mg of the compound (1a-1) as a photocatalyst, and 84.9 mg of ammonium oxalate as a reducing agent were charged into a reaction vessel, 1.1 mL of water and 2.1 mL of a compound (13-2) as a carbonyl compound (organic compound (II)) were added thereto, and then the mixture was stirred at 40°C for 24 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-8).

The yield of the obtained compound (P-8) was determined by NMR measurement. The results are shown in Table 3.

### Example 10

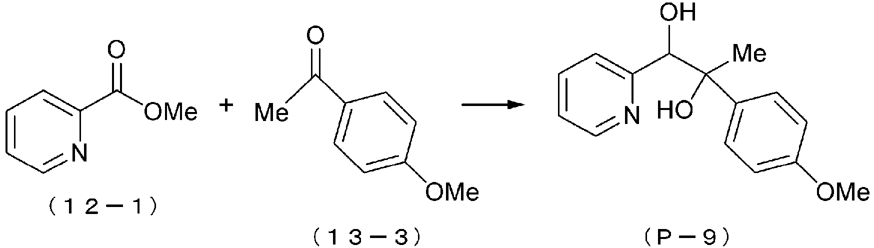

26.7 mg of the compound (12-1) as an ester compound (organic compound (I)), 149 mg of the compound (13-3) as a carbonyl compound (organic compound (11)), 3.36 mg of the compound (1a-1) as a photocatalyst, and 85.0 mg of ammonium oxalate as a reducing agent were charged into a reaction vessel, 1.6 mL of water and 1.6 mL of acetonitrile were added thereto, and then the mixture was stirred at 40°C for 24 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-9).

The yield of the obtained compound (P-9) was determined by NMR measurement. The results are shown in Table 3.

### Example 11

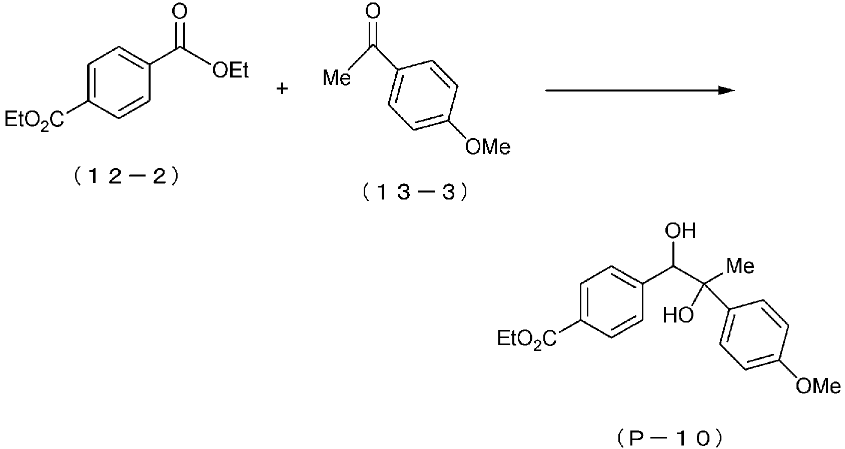

44.3 mg of a compound (12-2) as an ester compound (organic compound (I)), 151 mg of a compound (13-3) as a carbonyl compound (organic compound (11)), 3.35 mg of the compound (1a-1) as a photocatalyst, and 285 mg of ammonium oxalate as a reducing agent were charged into a reaction vessel, 1.6 mL of water and 1.6 mL of acetonitrile were added thereto, and then the mixture was stirred at 50°C for 24 hours while irradiating with blue light (manufactured by Kessil, product name "A160WE Tuna Blue", output 40 W, wavelength 462 nm). After cooling, a saturated sodium chloride aqueous solution and ethyl acetate were added thereto, the organic layer was extracted, and the extracted organic layer was purified by column chromatography and dried to obtain the following compound (P-10).

The yield of the obtained compound (P-10) was determined by NMR measurement. The results are shown in Table 3.

**[Table 3]**

| | Organic compound (I) | Organic compound (II) | Photocatalyst | Yield [%] |
|---|---|---|---|---|
| Example 8 | Compound (12-1) | Compound (13-1) | Compound (1a-1) | 43 |
| Example 9 | Compound (12-1) | Compound (13-2) | Compound (1a-1) | 62 |
| Example 10 | Compound (12-1) | Compound (13-3) | Compound (1a-1) | 46 |
| Example 11 | Compound (12-2) | Compound (13-3) | Compound (1a-1) | 65 |

As is clear from Table 3, with the polycyclic compound according to the embodiment of the present invention, visible light could be used as an energy source to couple an ester compound with a carbonyl compound other than the ester compound, and the target coupled product could be produced with a high yield.

### INDUSTRIAL APPLICABILITY

Since the polycyclic compound according to the embodiment of the present invention has high oxidizing power and reducing power and can use visible light as an energy source, it is suitable as a photocatalyst used in, for example, a reduction reaction or a coupling reaction. Moreover, the polycyclic compound according to the embodiment of the present invention is also suitable as a fluorescent material for, for example, an organic EL element, a wavelength conversion film, a fluorescent paint, and the like.

## Claims

1. A polycyclic compound represented by Formula (1a) or Formula (1b), 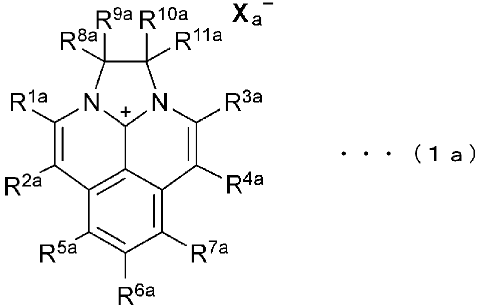 in Formula (1a), R^{1a} to R^{11a} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and Xₐ⁻ is a counter anion, 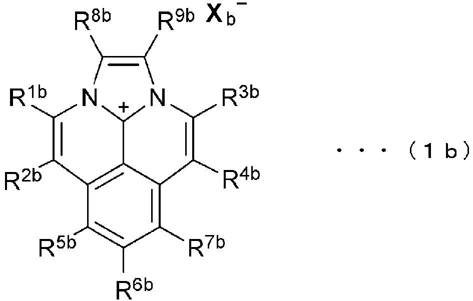 in Formula (1b), R^{1b} to R^{9b} are each independently a hydrogen atom, a hydroxy group, a hydrocarbon group having 1 to 20 carbon atoms and optionally containing a hetero atom, a hydrocarbonoxy group having 1 to 20 carbon atoms, a monoalkylamino group having 1 to 20 carbon atoms, a dialkylamino group having 2 to 21 carbon atoms, an ester group, or a cyano group, and X_{b}⁻ is a counter anion.

2. The polycyclic compound according to Claim 1,
wherein, in Formula (1a), R^{1a} to R^{4a} are each independently a hydrocarbon group having 1 to 20 carbon atoms and optionally containing a hetero atom, R^{6a} is a hydrogen atom, a hydroxy group, a hydrocarbonoxy group having 1 to 20 carbon atoms, or a dialkylamino group having 2 to 21 carbon atoms, and R^{5a}, R^{7a}, and R^{8a} to R^{11a} are each a hydrogen atom, and
in Formula (1b), R^{1b} to R^{4b} are each independently a hydrocarbon group having 1 to 20 carbon atoms and optionally containing a hetero atom, R^{6b} is a hydrogen atom, a hydroxy group, a hydrocarbonoxy group having 1 to 20 carbon atoms, or a dialkylamino group having 2 to 21 carbon atoms, and R^{5b}, R^{7b}, R^{8b}, and R^{9b} are each a hydrogen atom.

3. The polycyclic compound according to Claim 2,
wherein R^{1a} to R^{4a} in Formula (1a) are each independently an alkyl group having 1 to 20 carbon atoms and optionally containing a hetero atom or an aralkyl group having 1 to 20 carbon atoms and optionally containing a hetero atom, and
R^{1b} to R^{4b} in Formula (1b) are each independently an alkyl group having 1 to 20 carbon atoms and optionally containing a hetero atom or an aralkyl group having 1 to 20 carbon atoms and optionally containing a hetero atom.

4. The polycyclic compound according to Claim 3,
wherein R^{1a} to R^{4a} in Formula (1a) are each independently an alkyl group having 1 to 10 carbon atoms and containing no hetero atom, and
R^{1b} to R^{4b} in Formula (1b) are each independently an alkyl group having 1 to 10 carbon atoms and containing no hetero atom.

5. The polycyclic compound according to any one of Claims 1 to 4,
wherein the polycyclic compound is a photocatalyst.

6. The polycyclic compound according to any one of Claims 1 to 4,
wherein the polycyclic compound is a fluorescent material.

7. A method for reducing an organic compound, comprising reducing an organic compound using the polycyclic compound according to any one of Claims 1 to 4.

8. A method for coupling organic compounds, comprising coupling organic compounds using the polycyclic compound according to any one of Claims 1 to 4.
